# DEMANDE DE BREVET EUROPEEN

(11) **EP 1 878 468 A1**
(43) Date de publication de la demande: **16.01.2008**
(21) Numéro de dépôt: 07111729.5
(22) Date de dépôt: 04.07.2007
(51) Int. Cl.: A61Q 5/02, A61Q 5/12, A61K 8/73

(54) **Composition detergente comprenant un tensioactif non ionique et une gomme de gellane et utilisation**

(30) Priorité: 10.07.2006 FR 0652883
(71) Demandeur: L'Oréal, 75008 Paris (FR)
(72) Inventeur: PARRIS, Eric, 92110, CLICHY (FR); FACK, Géraldine, 92300, LEVALLOIS (FR)
(74) Mandataire: Le Blainvaux Bellegarde, Françoise

(57) **Abrégé**

La présente invention a pour objet d'une composition cosmétique détergente comprenant de 3 à 30% de gomme de gellane et au moins un tensioactif non ionique choisi parmi les alkylpolyglycosides et les tensioactifs mono ou polyglycérés.

Elle concerne également l'utilisation de cette composition détergente, pour le lavage des cheveux.

## Description

La présente invention a pour objet une composition pour application topique comprenant une gomme de gellane et un tensioactif non ionique particulier, un procédé de traitement de fibres kératiniques ainsi que son utilisation pour le lavage et le conditionnement des matières kératiniques, en particulier des fibres kératiniques, ainsi qu'un procédé de mise en oeuvre de ladite composition.

Plus précisément, le domaine de l'invention est celui du traitement des matières kératiniques, en particulier les fibres kératiniques notamment colorées, de préférence de fibres kératiniques humaines, comme notamment les cheveux.

Pour le nettoyage et/ou le lavage des cheveux, l'utilisation de compositions détergentes (ou shampooings) à base essentiellement d'agents tensioactifs classiques de type notamment anionique est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Les agents tensioactifs de la famille des alkylpolyglycosides ou polyglycérolés ont déjà été préconisés dans des compositions lavantes pour les cheveux ou la peau. Ce sont des détergents doux, bien tolérés et biodégradables.

Généralement, les compositions lavantes sont épaissies. On recherche des produits que l'on peut doser et prendre aisément dans la main. Pour ce faire, ces produits doivent présenter une certaine consistance ou viscosité. En effet, un produit liquide est beaucoup plus difficile à doser et s'écoule facilement entre les doigts, ce qui gêne l'application sur les cheveux. Par ailleurs, dans le cas d'un shampooing, le produit peut s'écouler sur le visage et en particulier dans les yeux, ce qui n'est pas souhaitable.

Cependant, l'épaississement des compositions à base de tensioactifs non ioniques est difficilement réalisé par les méthodes traditionnelles (amides de coprah, électrolytes..) en raison de leur structure non-ionique. Pour épaissir les compositions cosmétiques contenant ces agents tensioactifs, on a déjà utilisé des polymères synthétiques ou naturels tels que les polymères acryliques réticulés du type Carbopol, les celluloses... Malheureusement, parfois les épaississants donnent des compositions instables et/ou non transparentes. De plus, la plupart des agents épaississants présentent l'inconvénient de diminuer la qualité de la mousse et les performances cosmétiques des shampooings, notamment en rendant les cheveux plus chargés et plus rêches. La mousse de ces compositions épaissies n'est généralement pas suffisamment douce, elle ne se développe pas facilement que ce soit en vitesse ou en abondance.

Ainsi, il subsiste le besoin d'un système épaississant permettant d'épaissir, fortement une composition lavante comprenant des tensioactifs non ioniques sans dégrader les propriétés cosmétiques et moussantes desdites compositions.

Le but de la présente invention est d'obtenir des compositions détergentes des cheveux, ayant une viscosité élevée et donnant une mousse abondante, fine, et se développant facilement.

La demanderesse vient de découvrir, de manière surprenante, que l'association, dans des compositions lavantes pour matières kératiniques, de gommes de gellane à des agents tensioactifs non ioniques type alkylpolyglycoside et/ou polyglycérolé permettait d'obtenir des compositions stables, homogènes, esthétiques et présentant une texture épaisse. Par ailleurs, lors de l'application de la composition sur les cheveux mouillés, la mousse démarre facilement et la mousse est abondante et aérée. Ces propriétés permettent d'utiliser une quantité inférieure de shampooing pour un volume de mousse équivalent.

Par ailleurs, les propriétés de l'invention peuvent présenter une viscosité très élevée et/ou un aspect granité (tel que la glace pilée) particulièrement original au regard des produits cosmétiques classiques et en particulier dans le domaine des shampooings.

Ainsi, la présente invention a pour objet une composition cosmétique détergente comprenant dans un milieu aqueux notamment physiologiquement acceptable et en particulier cosmétiquement acceptable, de 1 à 30% en poids par rapport au poids total de la composition d'au moins un tensioactif non ionique choisi parmi les alkylpolyglycosides et les tensioactifs mono ou polyglycérolés et de 3 à 30% en poids par rapport au poids total de la composition d'au moins une gomme de gellane ou l'un de ses dérivés.

Un autre objet de l'invention est constitué par le procédé de lavage et/ou de conditionnement mettant en oeuvre de telles compositions.

Mais d'autres avantages et caractéristiques de l'invention apparaîtront plus clairement à la lecture de la description et des exemples qui vont suivre.

En ce qui concerne les alkypolyglycosides, ces composés sont bien connus et peuvent être plus particulièrement représenté par la formule générale suivante :

R₁O-(R₂O)ₜ(G)ᵥ (I)

dans laquelle R₁ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, R₂ représente un radical alkylène comportant environ de 2 à 4 atomes de carbone, G représente un motif sucre comportant de 5 à 6 atomes de carbone, t désigne une valeur allant de 0 à 10, de préférence 0 à 4, de préférence 0 à 4 et v désigne une valeur allant de 1 à 15.

Des alkylpolyglycosides préférés selon la présente invention sont des composés de formule (I) dans laquelle R₁ désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation, i.e. la valeur de v dans la formule (I), peut aller de 1 à 15, de préférence de 1 à 4. Le degré moyen de polymérisation est plus particulièrement compris entre 1 et 2 et encore plus préférentiellement de 1,1 à 1,5.
Les liaisons glycosidiques entre les motifs sucre sont de type 1-6 ou 1-4 et de préférence 1-4.

Des composés de formule (I) sont notamment représentés par les produits vendus par la société COGNIS sous les dénominations PLANTAREN^{®} (600 CS/U, 1200 et 2000) ou PLANTACARE^{®} (818, 1200 et 2000). On peut également utiliser les produits vendus par la société SEPPIC sous les dénominations TRITON CG 110 (ou ORAMIX CG 110) et TRITON CG 312 (ou ORAMIX^{®} NS 10), les produits vendus par la société B.A.S.F. sous la dénomination LUTENSOL GD 70 ou encore ceux vendus par la société CHEM Y sous la dénomination AG10 LK.
On peut également utiliser par exemple, l'Alkyl en C8/C16 polyglucoside 1,4 en solution aqueuse à 53% commercialisé par COGNIS sous la référence PLANTACARE^{®} 818 UP.

En ce qui concerne les tensioactifs mono ou polyglycérolés, ils comportent de préférence en moyenne de 1 à 30 groupements glycérol, plus particulièrement de 1 à 10 groupements glycérol et en particulier de 1,5 à 5.

Les tensioactifs monoglycérolés ou polyglycérolés sont de préférence choisis parmi les composés suivants :
A) les composés de formule suivantes : RO[CH₂CH(CH₂OH)O]ₘH , RO[CH₂CH(OH)CH₂O]ₘH ou RO[CH(CH₂OH)CH₂O]ₘH ; dans laquelle R représente un radical hydrocarboné saturé ou insaturé, linéaire ou ramifié, comportant de 8 à 40 atomes de carbone et de préférence de 10 à 30 atomes de carbone ; m est un nombre compris entre 1 et 30, de préférence entre 1 et 10, plus particulièrement de 1,5 à 5.

R peut éventuellement comprendre des hétéroatomes tels que par exemple oxygène et azote. En particulier, R peut éventuellement comprendre un ou plusieurs groupements hydroxy et/ou éther et/ou amide.

R désigne de préférence des radicaux alkyle et/ou alkényle en C10-C20, éventuellement mono ou polyhydroxylé.

On peut utiliser par exemple, l'hydroxylauryléther polyglycérolé (3.5 moles) commercialisé sous la dénomination Chimexane® NF de Chimex.

Le ou les tensioactifs non ioniques choisis parmi les alkylpolyglycosides et les tensioactifs mono ou polyglycérés sont préférence les alkylpolyglycosides et notamment les alkyl C6-C24 polyglucosides et plus particulièrement les alkyl C8-C16 polyglucosides.

Le ou les tensioactifs non ioniques choisis parmi les alkylpolyglycosides et les tensioactifs mono ou polyglycérés sont présents dans les compositions selon l'invention dans des concentrations allant de 1 à 30% en poids, plus particulièrement de 5 à 25 % en poids, de préférence de 8 à 20% en poids par rapport au poids total de la composition.

La gomme de gellane est un polysaccharide produit par fermentation aérobie de *Sphingomonas elodea,* plus communément nommé *Pseudomonas elodea.* Ce polysaccharide linéaire est constitué par l'enchaînement des monosaccharides suivants : D-Glucose, acide D-glucuronique et L-Rhamnose. A l'état natif, la gomme de gellane est hautement acylée.
La gomme de gellane utilisée de manière préférée dans les compositions selon la présente invention est une gomme de gellane au moins partiellement déacylée. Cette gomme de gellane au moins partiellement déacylée est par exemple obtenue par un traitement alcalin à haute température.
On utilisera par exemple une solution de KOH ou de NaOH.
Selon l'invention, on utilise la gomme de gellane purifiée déacylée vendue sous la dénomination commerciale « KELCOGEL® » par la société CP KELCO convient pour préparer les compositions selon l'invention, en particulier sous la dénomination KELCOGEL F.
Les dérivés de la gomme de gellane sont tous les produits obtenus en mettant en oeuvre des réactions chimiques classiques, tels que notamment les estérifications, addition d'un sel d'un acide organique ou minéral.
A titre de dérivé de la gomme de gellane, on utilise par exemple la gomme de welane. La gomme de welane est une gomme de gellane modifiée par fermentation au moyen de *Alcaligenes* souche ATCC 31 555. La gomme de welane possède une structure pentasaccharidique récurrente formée d'une chaîne principale constituée d'unités D-Glucose, acide D-glucuronique et L-Rhamnose sur laquelle un motif pendant de L-Rhamnose ou de L-Mannose est greffé.
La gomme de welane vendue sous la dénomination commerciale « KELCO CRETE ®» par la société KELCO convient pour préparer les compositions selon l'invention.
De préférence, on utilisera une gomme de gellane.

La concentration en gomme de gellane ou ses dérivés utilisée dans les compositions selon la présente invention est comprise de préférence dans des proportions allant de 5 à 20%, plus particulièrement de 8 à 15% en poids et encore plus particulièrement de 8 à 12% en poids par rapport au poids total de la composition.

Les compositions selon l'invention peuvent comprendre en outre des tensioactifs additionnels tels que des tensioactifs anioniques, amphotères, zwittérioniques et leurs mélanges. Les compositions peuvent également comprendre des tensioactifs non ioniques différents des tensioactifs non ioniques choisis parmi les alkylpolyglycosides et les tensioactifs mono ou polyglycérolés.

Pour ce qui a trait aux tensioactifs amphotères ou zwittérioniques, on peut mentionner sans avoir l'intention de s'y limiter, les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives : R₂ -CONHCH₂CH₂ -N(R₃)(R₄)(CH₂COO⁻)
dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₂'-CONHCH₂CH₂-N(B)(C)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂-CHOH-SO₃H
R₂' désigne un radical alkyle d'un acide R₉-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylampho-dipropionate, Disodium Capryloamphodipropionate, Lauroamphodipropionic acid, Cocoamphodipropionic acid, disodium cocoamphocarboxy ethyl hydroxypropyl sulfonate.
A titre d'exemple on peut citer le cocoamphodiacetate commercialisé sous la dénomination commerciale MIRANOL^{®} C2M concentré par la société Rhodia Chimie.

Les composition selon l'invention peuvent également contenir des tensioactifs anioniques et plus particulièrement des tensioactifs anioniques doux.

A titre d'exemple de tensio-actifs anioniques utilisables, seuls ou en mélange, dans le cadre de la présente invention, on peut citer notamment les sels (en particulier sels alcalins, notamment sels de sodium, sels d'ammonium, sels d'amines, sels d'aminoalcools ou sels de magnésium) des composés suivants : les alkylsulfates, alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, alkyléthersulfosuccinates, alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle.

Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D-galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (II) suivante :

R₁-(OC₂H₄)ₙ- OCH₂COOA (II)

dans laquelle :
- R₁ désigne un groupement alkyle, alkylamido ou alkaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
- A désigne H, ammonium, Na, K, Li, Mg ou un reste monoéthanolamine ou triéthanolamine. On peut également utiliser des mélanges de composés de formule (II) en particulier des mélanges dans lesquels les groupements R₁ sont différents.

Des composés de formule (II) sont vendus par exemple par la Société CHEM Y sous les dénominations AKYPO (NP40, NP70, OP40, OP80, RLM25, RLM38, RLMQ 38 NV, RLM 45, RLM 45 NV, RLM 100, RLM 100 NV, RO 20, RO 90, RCS 60, RS 60, RS 100, RO 50) ou par la Société SANDOZ sous les dénominations SANDOPAN (DTC Acid, DTC).

Les tensioactifs anioniques convenant plus particulièrement aux compositions selon l'invention sont choisis parmi le lauryléther sulfate de sodium ou d'ammonium, le lauryl sulfate de sodium ou d'ammonium et/ou leurs mélanges.

En ce qui concerne les tensioactifs anioniques doux, on peut citer notamment les composés suivants et leurs sels, ainsi que leurs mélanges :
- les acides alkyl éther carboxyliques polyoxyalkylénés,
- les acides alkylaryl éther carboxyliques polyoxyalkylénés,
- les acides alkylamido éther carboxyliques polyoxyalkylénés en particulier ceux comportant 2 à 50 groupements oxyde d'éthylène,
- les acides d'alkyl D galactoside uroniques
- les acylsarcosinates, les acylglutamates ;
- les esters d'alkylpolyglycosides carboxyliques ;

Tout particulièrement, on utilise des acides alkyl éther carboxyliques polyoxyalkylénés comme par exemple l'acide lauryl ether carboxylique (4,5 OE) commercialisé par exemple sous la dénomination AKYPO RLM 45 CA de KAO.

Si de tels tensioactifs additionnels sont présents, alors la teneur en tensioactif(s) additionnel(s) va de 0,1 à 20% en poids, par rapport au poids total de la composition, plus particulièrement de 1 % à 10 % en poids, par rapport au poids total de la composition.

Les compositions selon l'invention comprennent de préférence une concentration totale en tensioactifs allant de 4 à 50% en poids, plus particulièrement de 8 à 30% en poids par rapport au poids total de la composition.

Les compositions selon l'invention peuvent de plus comprendre les additifs classiques dans le domaine comme par exemple ceux choisis parmi la liste non exhaustive tels que les agents réducteurs, les agents oxydants, les séquestrants, les adoucissants, les agents anti-mousse, les agents hydratants, les agents émollients, les agents alcalinisants, les plastifiants, les filtres solaires, les colorants directs ou d'oxydation, les pigments, les charges minérales, les argiles, les minéraux colloïdaux, les nacres, les agents nacrant, les parfums, les peptisants, les conservateurs, les polymères fixant ou non, les protéines, les vitamines, les agents antipelliculaires, les agents antiséborrhéïques, les agents antichute des cheveux, les acides gras, les polymères épaississants associatifs ou non, les amides grasses, les alcools gras, etc.

Les adjuvants cités ci-dessus sont en général présents en quantité comprise, pour chacun d'eux, entre 0,01 et 20% en poids par rapport au poids de la composition.
Il est à noter que si la composition comprend un ou agents épaississants, leur teneur est comprise entre 0,01 et 20% en poids par rapport au poids de la composition, de préférence de 0,01 à 3% en poids par rapport au poids de la composition.

De préférence, la composition selon l'invention comprend au moins un agent conditionneur.
Lorsque la composition contient au moins un agent de conditionnement, ils sont généralement choisis parmi les huiles de synthèse telles que les poly-α-oléfines, les huiles fluorées, les cires fluorées, les gommes fluorées, les cires non fluorées, les esters gras d'acides carboxyliques, les polymères cationiques, les silicones, les huiles minérales, végétales ou animales, les céramides, les pseudocéramides et leurs mélanges.

Selon un mode de réalisation préféré, le ou les agents conditionneurs sont choisis parmi les polymères cationiques, les silicones, et leurs mélanges. De préférence l'agent conditionneur est choisi parmi les silicones, de préférence non volatiles.

Selon ce mode de réalisation, la teneur en agent conditionneur dans la composition selon l'invention est comprise de 0,001 % à 25 % en poids, de préférence de 0,005 % à 10 % en poids, et encore plus préférentiellement de 0,01 % à 5 % en poids, du poids total de la composition finale.

La composition selon l'invention peut comprendre en outre un ou plusieurs polymères cationiques. Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

Au sens de la présente invention, l'expression "polymère cationique" désigne tout polymère comprenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français 2 505 348 ou 2 542 997. Parmi ces polymères, on peut citer :
- les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
- les copolymères d'acrylamide et de chlorure de méthacryloyloxyéthyl-triméthylammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxy-éthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français FR 2 077 143 et 2 393 573,
- les terpolymères méthacrylate de diméthyl amino éthyle/vinylcaprolactame/ vinylpyrrolidone tel que le produit vendu sous la dénomination GAFFIX VC 713 par la société ISP,
- les copolymères vinylpyrrolidone / méthacrylamidopropyl dimethylamine commercialisés notamment sous la dénomination STYLEZE CC 10 par ISP.
- et les copolymères vinylpyrrolidone / méthacrylamide de diméthyl-aminopropyle quaternisé tel que le produit vendu sous la dénomination "GAFQUAT HS 100" par la société ISP.

Conviennent également à titre de polymères cationiques, les polysaccharides cationiques notamment les celluloses à l'image par exemple des polymères commercialisés sous les dénominations "JR" (JR 400, JR 125, JR 30M) ou "LR" (LR 400, LR 30M) par la Société AMERCHOL et les produits commercialisés sous la dénomination "Celquat L 200" et "Celquat H 100" par la Société National Starch et les gommes de galactomannanes cationiques comme par exemple les produits commercialisés notamment sous les dénominations commerciales de JAGUAR C13 S, JAGUAR C 15, JAGUAR C 17 ou JAGUAR C162 par la société RHODIA.

Les polymères cationiques convenant à l'invention peuvent également être choisis parmi :
- les dérivés de polyaminoamides comme par exemple les polymères acide adipique/diméthylaminohydroxypropyl/diéthylène triamine vendus sous la dénomination "Cartaretine F, F4 ou F8" par la société Sandoz,
- les polymères dérivant de polyalkylène polyamine comme par exemple ceux commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
- les cyclopolymères d'alkyl diallyl amine ou de dialkyl diallyl ammonium tels que les polymères vendus sous la dénomination "MERQUAT 100" par la société NALCO (et leurs homologues de faibles masses molaires moyenne en poids) et les copolymères de sels (par exemple chlorure) de diallyldiméthylammonium et d'acrylamide par exemple commercialisés sous la dénomination "MERQUAT 550",
- les polymères de diammonium ou de polyammonium quaternaires comme par exemple les produits "Mirapol® A 15", "Mirapol® AD1", "Mirapol® AZ1" et "Mirapol® 175" vendus par la société Miranol,
- les polymères quaternaires de vinylpyrrolidone et de vinylimidazole tels que par exemple les produits commercialisés sous les dénominations Luviquat^{®} FC 905, FC 550 et FC 370 par la société B.A.S.F,
- les polyamines comme le Polyquart^{®} H vendu par COGNIS, référencé sous le nom de "POLYETHYLENEGLYCOL (15) TALLOW POLYAMINE" dans le dictionnaire CTFA, et
- les polymères de préférence réticulés de sels de méthacryloyloxyalkyl(C₁-C₄) trialkyl(C₁-C₄)ammonium tels que par exemple les produits commercialisés à l'état de dispersion sous le nom de "SALCARE^{®} SC 92", de "SALCARE^{®} SC 95" et "SALCARE^{®} SC 96" par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on peut utiliser de préférence les dérivés d'éther de cellulose quaternaires tels que les produits vendus sous la dénomination "JR 400" par la Société AMERCHOL, les cyclopolymères cationiques, en particulier les homopolymères ou copolymères de sels (par exemple chlorure) de diméthyldiallylammonium, vendus sous les dénominations "MERQUAT 100", "MERQUAT 550" et "MERQUAT S" par la société NALCO et leurs homologues de faibles poids moléculaires en poids, les polysaccharides cationiques tels que les gommes de guar modifiées par un sel de 2,3-époxypropyl triméthylammonium, les polymères quaternaires de vinylpyrrolidone et de vinylimidazole, les homopolymères ou copolymères éventuellement réticulés de sels de méthacryloyloxyalkyl(C₁-C₄) trialkyl(C₁-C₄)ammonium et leurs mélanges.

Le ou les polymères cationiques sont généralement présents à des concentrations allant de 0,01 à 20 %, de préférence de 0,05 à 10 % et plus particulièrement de 0,1 à 5 % en poids par rapport au poids total de la composition.

Les compositions selon l'invention comprennent de préférence au moins une silicone.

A titre de silicones utilisables dans les compositions de la présente invention, on peut notamment citer les silicones volatiles ou non, cycliques ou acycliques, ramifiées ou non, organomodifiées ou non, telles que décrites ci-dessous.

Les silicones utilisables conformément à l'invention peuvent être solubles ou insolubles dans la composition et en particulier être des polyorganosiloxanes insolubles dans la composition de l'invention ; elles peuvent se présenter sous forme d'huiles, de cires, de résines ou de gommes.

Selon l'invention, toutes les silicones peuvent être utilisées telle quelle ou sous forme de solutions, de dispersions, d'émulsions, de nanoémulsions ou de microémulsions.

Les organopolysiloxanes sont définis plus en détail dans l'ouvrage de Walter NOLL "Chemistry and Technology of Silicones" (1968) Academie Press. Elles peuvent être volatiles ou non volatiles.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25 °C et de préférence 1.10⁻⁵ à 1 m²/s. La viscosité des silicones est, par exemple, mesurée à 25 °C selon la norme ASTM 445 Appendice C.

Parmi ces polyalkylsiloxanes, on peut citer à titre non limitatif les produits commerciaux suivants :
- les huiles SILBIONE^{®} des séries 47 et 70 047 ou les huiles MIRASIL^{®} commercialisées par RHODIA telles que, par exemple, l'huile 70 047 V 500 000 ;
- les huiles de la série MIRASIL^{®} commercialisées par la société RHODIA ;
- les huiles de la série 200 de la société DOW CORNING telles que plus particulièrement la DC200 de viscosité 60 000 mm²/s ;
- les huiles VISCASIL^{®} de GENERAL ELECTRIC et certaines huiles des séries SF (SF 96, SF 18) de GENERAL ELECTRIC.

On peut également citer les polydiméthylsiloxanes à groupements terminaux diméthylsilanol, connus sous le nom de dimethiconol (CTFA), tels que les huiles de la série 48 de la société RHODIA .

Dans cette classe de polyalkylsiloxanes, on peut également citer les produits commercialisés sous les dénominations "ABIL WAX^{®} 9800 et 9801" par la société GOLDSCHMIDT qui sont des polyalkyl(C₁-C₂₀)-Siloxanes.

Il peut s'agir de polyalkylarylsiloxanes, notamment choisis parmi les polydiméthyl/méthylphénylsiloxanes, les polydiméthyldiphényl-siloxanes linéaires et/ou ramifiés de viscosité de 1.10⁻⁵ à 5.10⁻² m²/s à 25 °C.

Parmi ces polyalkylarylsiloxanes on peut citer, à titre d'exemple, les produits commercialisés sous les dénominations suivantes :
- les huiles SILBIONE^{®} de la série 70 641 de RHODIA ;
- les huiles des séries RHODORSIL^{®} 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Des produits plus particulièrement utilisables conformément à l'invention sont des mélanges tels que :
- les mélanges formés à partir d'un polydiméthylsiloxane hydroxylé en bout de chaîne ou diméthiconol (CTFA) et d'un poly-diméthylsiloxane cyclique également appelé cyclométhicone (CTFA) tel que le produit Q2 1401 commercialisé par la société DOW CORNING ;
- les mélanges formés à partir d'une gomme polydiméthyl-siloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶ _{M}²/_{S}. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SiO_{1/2}, RSiO_{3/2} et SiO_{4/2} dans lesquels R représente un groupement hydrocarboné possédant 1 à 16 atomes de carbone ou un groupement phényle. Parmi ces produits, ceux particulièrement préférés sont ceux dans lesquels R désigne un groupe alkyle inférieur en C₁-C₄, plus particulièrement méthyle, ou un groupe phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthylsiloxane.

On peut également citer les résines du type triméthylsiloxysilicate commercialisées notamment sous les dénominations X22-4914, X21-5034 et X21-5037 par la société SHIN-ETSU.

Les silicones telles que décrites ci-dessus peuvent être utilisées seules ou en mélange, en une quantité comprise entre 0,01 et 20 % en poids, de préférence entre 0,1 et 5 % en poids par rapport au poids total de la composition.

Le milieu aqueux notamment physiologiquement acceptable pour les matières kératiniques est généralement constitué par de l'eau ou par un mélange d'eau et d'au moins un solvant organique acceptable sur le plan cosmétique.
A titre de solvant organique, on peut par exemple citer les monoalcools, linéaires
ou ramifiés, de préférence saturés, comprenant 2 à 10 atomes de carbone, tels que l'alcool éthylique, l'alcool isopropylique ; les alcools aromatiques tels que l'alcool benzylique, l'alcool phényléthylique ; les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol, l'hexylèneglycol (2-méthyl 2,4-pentanediol), le néopentylglycol et le 3-méthyl-1,5-pentanediol ; ainsi que les alkyléthers de diéthylèneglycol, notamment en C₁-C₄, comme par exemple, le monoéthyléther ou le monobutyléther du diéthylèneglycol, seuls ou en mélange.

L'eau représente de préférence de 30 à 96% en poids et de préférence de 50 à 90% en poids et plus particulièrement de 70 à 85% en poids par rapport au poids total de la composition.

Les solvants peuvent être présents dans des proportions de préférence comprises entre 1 et 40 % en poids environ par rapport au poids total de la composition détergente, et encore plus préférentiellement entre 5 et 30 % en poids environ.

Selon l'invention, les compositions sont moussante, c'est-à-dire que le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée. Les modifications de la méthode sont les suivantes :
La mesure se fait à la température de 22°C avec de l'eau osmosée. La concentration de la solution est de 2g/l. La hauteur de la chute est de 1 m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

La composition selon l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures éventuellement halogénés (fluorés en particuliers) ou non et leurs mélanges.

Les compositions peuvent se présenter sous différentes formes galéniques tels qu'une lotion, un gel, un spray, une mousse conditionnée en aérosol ou dans un flacon-pompe, etc.

L'invention a encore pour objet un procédé de lavage des matières kératiniques telles que les cheveux consistant à appliquer sur celles-ci une composition telle que définie précédemment puis à effectuer éventuellement un rinçage à l'eau, après un éventuel temps de pause.

Les compositions selon l'invention peuvent être préparées en mélangeant à chaud (par exemple 60-80°C) tous les ingrédients à l'exception de la gomme de gellane jusqu'à homogénéisation complète du milieu. On laisse refroidir le milieu sous agitation jusqu'à température ambiante. On introduit alors la gomme de gellane sous agitation jusqu'à homogénéisation complète de la composition.

Des exemples concrets mais non limitatifs de l'invention vont maintenant être présentés.

### EXEMPLES 1 ET 2

On a préparé les 2 compositions de shampooing suivantes :

| | 1 | 2 |
|---|---|---|
| Décyl (C9-C11)polyglucoside (1,4) à 40% dans l'eau (MYDOL 10 de KAO) | 10gMA | 10gMA |
| Polyquaternium-10 (JR400-Amerchol) | | 0,5gMA |
| Gomme de gellane (KELCOGEL F-CP de KELCO | 10g | 10 g |
| Parfum, conservateurs | qs | qs |
| Agent de pH qs | pH 7 ± 0,5 | pH 7 ± 0,5 |
| Eau qsp | 100 g | 100 g |

La qualité de la mousse des formules a été évaluée sur des mèches de cheveux (vitesse de démarrage, l'abondance et la tenue)

Les compositions 1 et 2 présentent un épaississement suffisant, stable, et de bonnes propriétés moussantes et cosmétiques.
Les compositions ont un aspect granité tel que la glace finement pilée.

### EXEMPLES 3 ET 4

On a préparé les 2 compositions de shampooing suivantes :

| | 3 | 4 |
|---|---|---|
| Décyl (C9-C11)polyglucoside (1,4) à 40% dans l'eau (MYDOL 10 de KAO) | 10gMA | 10gMA |
| Polyquaternium-10 (JR400-Amerchol) | 0,5 gMA | |
| Polyquaternium-6 (MERQUAT 100 de NALCO) | | 0,5gMA |
| Gomme de gellane (KELCOGEL F-CP de KELCO | 11 g | 10 g |
| Silicone aminée (DC939 emulsion de DOW CORNING) | 2,1 g MA | |
| Polydiméthylsiloxane en émulsion non ionique (DC2-1691 emulsion de DOW CORNING) | | 3 gMA |
| Parfum, conservateurs | qs | qs |
| Agent de pH qs | pH 7 ± 0,5 | pH 7 ± 0,5 |
| Eau qsp | 100 g | 100 g |

### EXEMPLES 5 ET 6

On a préparé les 2 compositions de shampooing suivantes :

| | 5 | 6 |
|---|---|---|
| Cocoglucoside (1,4) à 53% dans l'eau (PLANTACARE 818 UP de COGNIS) | 15gMA | 10gMA |
| Polyquaternium-10 (JR400-Amerchol) | 0,5 gMA | |
| Polyquaternium-6 (MERQUAT 100 de NALCO) | | 0,5gMA |
| Gomme de gellane (KELCOGEL F-CP de KELCO | 8g | 10 g |
| Parfum, conservateurs | qs | qs |
| Agent de pH qs | pH 7 ± 0,5 | pH 7 ± 0,5 |
| Eau qsp | 100 g | 100 g |

Les compositions des exemples 3 à 6 conduisent à des shampooings présentant une texture comparables à de la glace finement pilée.

## Revendications

1. Composition cosmétique détergente comprenant dans un milieu aqueux physiologiquement acceptable et en particulier cosmétiquement acceptable, de 1 à 30% en poids par rapport au poids total de la composition d'au moins un tensioactif non ionique choisi parmi les alkylpolyglycosides et les tensioactifs mono ou poly glycérolés, et de 3 à 30% en poids par rapport au poids total de la composition d'au moins une gomme de gellane ou l'un de ses dérivés,
la concentration totale en tensioactifs allant de 5 à 50% en poids par rapport au poids total de la composition.

2. Composition cosmétique selon la revendication 1 telle que le dérivé de gomme de gellane est une gomme de welane.

3. Composition cosmétique selon l'une des revendications précédentes telle que la concentration en gomme de gellane ou ses dérivés varie de 5 à 20% en poids, de préférence de 8 % 15%, en poids par rapport au poids total de la composition.

4. Composition selon l'une quelconque des revendications 1 à 3, **caractérisé en ce** les alkylpolyglucosides sont représenté par la formule générale suivante :
R₁O(R₂O)ₜ (G)v (I)
dans laquelle R₁ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, R₂ représente un radical alkylène comportant environ de 2 à 4 atomes de carbone, G représente un sucre comportant de 5 à 6 atomes de carbone, t désigne une valeur allant de 0 à 10, de préférence 0 à 4, et v désigne une valeur allant de 1 à 15.

5. Composition selon la revendication précédente, **caractérisée en ce que** dans la formule (I) R désigne un radical alkyle saturé ou insaturé, linéaire ou ramifié comportant de 8 à 18 atomes de carbone, t désigne une valeur égale à 0, G désigne le glucose, le fructose ou le galactose, v est une valeur allant 1 à 4.

6. Composition selon l'une des revendications 4 ou 5, **caractérisée en ce que** dans la formule (I), G désigne le glucose et v est une valeur allant de 1,1 à 2.

7. Composition selon l'une quelconque des revendications 1 à 6, **caractérisé en ce que** les tensioactifs monoglycérolés ou polyglycérolés sont de préférence choisis parmi les composés suivants :
A) les composés de formule suivantes : RO[CH₂CH(CH₂0H)O]ₘH , RO[CH₂CH(OH)CH₂O]ₘH ou RO[CH(CH₂OH)CH₂O]ₘH ; dans laquelle R représente un radical hydrocarboné saturé ou insaturé, linéaire ou ramifié, comportant de 8 à 40 atomes de carbone et de préférence de 10 à 30 atomes de carbone ; m est un nombre compris entre 1 et 30, de préférence entre 1 et 10.

8. Composition selon la revendication 7, **caractérisé en ce que** R désigne de préférence des radicaux alkyle et/ou alkényle en C10-C20, éventuellement mono ou polyhydroxylé.

9. Composition selon la revendication 7, **caractérisé en ce que** R peut comprendre des hétéroatomes tels que par exemple oxygène et azote.

10. Composition selon l'une des revendications précédentes, **caractérisée en ce que** le ou les tensioactifs non ioniques sont présents dans les compositions selon l'invention dans des concentrations allant de 5 à 25 % en poids par rapport au poids total de la composition.

11. Composition selon l'une des revendications précédentes, **caractérisée en ce que** la composition comprend en outre des tensioactifs additionnels tels que des tensioactifs anioniques, amphotères, zwittérioniques et leurs mélanges.

12. Composition selon la revendication précédente, **caractérisée en ce que** la teneur en tensioactif(s) additionnel(s) va de 0,1 à 20% en poids, par rapport au poids total de la composition, plus particulièrement de 1% à 10 % en poids, par rapport au poids total de la composition.

13. Composition selon l'une quelconque des revendications précédentes, **caractérisée en ce qu'**elle comprend en outre au moins un agent conditionneur.

14. Composition selon la revendication précédente, **caractérisée en ce que** le ou les agents conditionneurs sont choisis parmi les poly-α-oléfines, les huiles fluorées, les cires fluorées, les gommes fluorées, les esters d'acides carboxyliques, les silicones, les polymères cationiques, les huiles minérales, végétales ou animales, les céramides, les pseudocéramides, et leurs mélanges.

15. Composition selon l'une quelconque des revendications 13 ou 14, **caractérisé en ce que** le ou les agents conditionneurs sont choisis parmi les polymères cationiques, les silicones et leurs mélanges.

16. Composition selon la revendication précédente, **caractérisé en ce que** l'agent conditionneur est une silicone.

17. Composition selon l'une quelconque des revendications 13 à 15, **caractérisé en ce que** l'agent conditionneur est un polymère cationique.

18. Composition selon l'une quelconque des revendications 13 à 17, **caractérisée en ce** les agents conditionneurs représentent de 0,001 % à 25 % en poids, de préférence de 0,005 % à 10 % en poids, par rapport au poids total de la composition.

19. Procédé de lavage des matières kératiniques en particulier des cheveux, **caractérisé par le fait qu'**il consiste à appliquer sur lesdites matières une composition telle que définie selon l'une quelconque des revendications 1 à 18 puis à effectuer éventuellement un rinçage à l'eau.
